# EUROPÄISCHE PATENTSCHRIFT

(11) **EP 0 888 080 B1**
(45) Veröffentlichungstag und Bekanntmachung des Hinweises auf die Patenterteilung: **11.08.1999**
(21) Anmeldenummer: 97914263.5
(22) Anmeldetag: 18.03.1997
(51) Int. Cl.: A61B 5/0215

(54) **DRUCKMESSVORRICHTUNG ZUR INVASIVEN BLUTDRUCKMESSUNG**
PRESSURE-MEASURING DEVICE FOR INVASIVE BLOOD PRESSURE MEASUREMENT
SYSTEME DE MESURE DE LA PRESSION POUR MESURE INVASIVE DE LA PRESSION SANGUINE

(30) Priorität: 19.03.1996 DE 19610828; 12.11.1996 DE 19646701
(43) Veröffentlichungstag der Anmeldung: 07.01.1999
(73) Patentinhaber: Petermeyer, Michael, 35274 Kirchhain-Schönbach (DE)
(72) Erfinder: Petermeyer, Michael, 35274 Kirchhain-Schönbach (DE)
(74) Vertreter: Schoppe, Fritz, Dipl.-Ing.
(86) Internationale Anmeldenummer: EP9701355
(87) Internationale Veröffentlichungsnummer: WO9734523

(56) Entgegenhaltungen:
- US-A- 4 817 629
- US-A- 4 825 876
- US-A- 5 273 047
- US-A- 5 335 551

## Beschreibung

Die vorliegende Erfindung betrifft eine Druckmeßvorrichtung zur invasiven Blutdruckmessung. Insbesondere befaßt sich die vorliegende Erfindung mit einer Vorrichtung zur direkten Messung von Blutdruckwerten, bei der ein Drucksensor von dem zu messenden Medium, insbesondere von dem Blut des zu untersuchenden Patienten kontaminationssicher getrennt ist.

Die direkte, invasive Blutdruckmessung wird seit langer Zeit praktiziert. Während Operationen oder auf Intensivstationen in Kliniken ist die direkte Messung von Blutdruckwerten, wie beispielsweise des arteriellen und des venösen Blutdrucks, ein wichtiger Bestandteil der Überwachung des Patienten. Hierzu wird ein Katheter, der das Drucksignal aufnimmt, über einen mit einer Kochsalzlösung gefüllten Schlauch mit einem Druckwandler verbunden, der den Druck in ein elektrisches Signal umsetzt. Typischerweise wird bei einer derartigen Messung ein externer Nullpunkt festgelegt, welcher sich in der Höhe des rechten Vorhofes des Herzens des Patienten befindet. Zu diesem Zweck wird der Druckwandler auf der Höhe des rechten Vorhofes befestigt und der auf den Druckwandler einwirkende Atmosphärendruck auf Null gesetzt. Details dieser Meßtechnik sind an sich bekannt und bedürfen daher keiner weitergehenden Erläuterung.

Für die invasive Blutdruckmessung werden heute zwei unterschiedliche Durckaufnehmersysteme eingesetzt, nämlich einerseits wiederverwendbare Blutdruckmeßvorrichtungen bzw. Transducer und nur einmal verwendbare Blutdruckmeßvorrichtungen, die auch als Einmal-Transducer bezeichnet werden.

Eine wiederverwendbare Blutdruckmeßvorrichtung bzw. ein wiederverwendbarer Transducer besteht typischerweise aus einem elektromechanischen Druckwandler, der beliebig oft verwendet werden kann und aus einem sogenannten Einmal-Dom, der mit dem elektromechanischen Druckwandler lösbar verbunden werden kann. Der Einmal-Dom steht über eine Schlauchleitung, über die er mit einer Kochsalzlösung befüllt werden kann, mit dem Katheter in Verbindung. Der Einmal-Dom hat eine integrierte, dünnwandige, typischerweise aus Kunststoff bestehende Membrane, die zur kontaminationssicheren Trennung von dem Druckwandler dient und gegen diesen in den meisten Fällen erst dann anliegt, wenn das System mit Druck beaufschlagt wird. Dadurch dehnt sich die Membrane aus und steht in Kontakt zum Druckwandler.

Bei dem sogenannten Einmal-Transducer ist der Druckaufnehmer in Form eines Sensorchips direkt in ein Kunststoffgehäuse eingebettet und mit einer Kunststoffschicht, die typischerweise aus Silikon besteht, überzogen. Die Druckübertragung erfolgt bei derartigen Einmal-Transducern über die Kochsalzlösung, die in direktem Kontakt zu der Silikonschicht steht.

Die widerverwendbaren Transducer und die Einmal-Transducer haben systemimmanente Vorteile und Nachteile, die die Wahl des geeigneten Transducers für den jeweiligen Meßzweck bestimmen.

Beim Gebrauch von mehrfach verwendbaren Transducern muß, wie oben erläutert wurde, lediglich der Einmal-Dom gewechselt werden. Da der elektromechanische Druckwandler wiederverwendet werden kann, trägt dieses Konzept zur Vermeidung von unerwünschtem Elektroschrott bei. Nötigerweise hat jedoch dieses System Kanten im Übergangsbereich von dem Einmal-Dom zu der Kunststoffmembran, die eine luftblasenfreie Befüllung des kompletten Druckübertragungssystems in der Praxis in der Regel verhindern. Luftblasen innerhalb des Einmal-Domes tragen jedoch in erheblichem Maße zu einer Dämpfung der Drucksignale bei, die zu falschen Messungen und somit zu falschen Therapien führen können.

Eine weitere Fehlerquelle der bekannten Systeme der wiederverwendbaren Transducer besteht darin, daß für einen ungestörten Meßvorgang eine glatte Ankoppelung der Trennmembran des Einmal-Domes an die Sensormembran des Druckwandlers gewährleistet sein muß. Aufgrund von Toleranzen bei der Herstellung der Einmal-Dome, die typischerweise Spritzgußartikel sind, ist eine derart glatte Ankopplung nicht immer gewährleistet.

In Hinblick auf die meßtechnische Genauigkeit sind hier die Einmal-Transducer den wiederverwendbaren Transducern deutlich überlegen, da sie sich aufgrund der Tatsache einwandfrei entlüften lassen, daß die Flüssigkeit, in der der Druck gemessen wird, über die Silikonschicht in direktem Kontakt mit der Sensormembran steht. Jedoch geht die Verwendung von Einmal-Transducern auch mit Risiken und Nachteilen einher. Ein erster offenkundiger Nachteil besteht darin, daß die Verwendung derartiger Einmaltransducer nötigerweise zu einem hohen Müllaufkommen führt. Der Druckaufnehmer und die Kabel zum Monitor werden bei dem typischen klinischen Einsatz dem normalen Hausmüll zugeführt und können nicht recycled werden, da einerseits die Gefahr einer Sekundärkontamination gegeben wäre und andererseits bei den meisten Produkten der Drucksensorchip unlöslich mit dem Gehäuse verbunden ist.

Ein weiteres Problem bei der Verwendung von Einmal-Transducern ergibt sich aus dem auch für derartige Transducer erforderlichen Sterilisationsvorgang. Die Einmal-Transducer müssen vollständig mit Ethylenoxid sterilisiert werden. In der Endphase des Sterilisationsprozesses wird dieses Gas über ein Vakuum abgesaugt. Dabei können Partikel der Silikonschicht dislozieren, welche später in den Blutkreislauf des Patienten gelangen können. Die Sensormembran bzw. das Silikongel des Einweg-Transducers hat einen direkten Kontakt mit dem Patientenkreislauf, so daß eine Mehrfachanwendung dieser Transducersysteme nicht mögich ist. Einer erneute Sterilisation von Einweg-Transducern verbietet sich bei dem heutigen Stand des Wissens.
Verschiedene Ausführungsformen derartiger Transducer sind auch in der Patentliteratur beschrieben. So zeigt die deutsche Patentschrift DE 2930869 C2 einen wiederverwendbaren Transducer, bestehend aus einem Einmal-Dom in Form einer Druckmeßkapsel, welche eine planare kreisförmige flexible Membran aufweist, die bei Befestigung der Druckmeßkapsel gegenüber einem Gehäuse eines Druckwandlers gegen den Druckwandler anliegt.

Die französische Patentschrift FR 2,125,991 zeigt einen Transducer mit einem Gehäuseteil, welches einen Drucksensor umschließt. Das Gehäusteil ist von einer Membran überspannt, die auf dem Drucksensor aufliegt. Ein zweites Geäuseteil, welches einen Hohlraum oberhalb der Membran bildet, ist gegenüber dem ersten Gehäuseteil verschraubbar oder mittels eines Schnappverschlusses in Eingriff bringbar. Die Flüssigkeit zur Druckübertragung steht bei dieser Druckmeßvorrichtung also direkt mit dem Membranbereich des Drucksensors in Verbindung, so daß nach einer Messung dieser sterilisiert werden muß.

Eine in der Praxis verbreitete Form eines wiederverwendbaren Transducers ist aus der US 4,539,998 A bekannt. Dieser bekannte wiederverwendbare Transducer für die invasive Blutdruckmessung umfaßt ein Gehäuse mit zwei Kammern, zwischen denen sich eine isolierende Zwischenwand erstreckt, wobei eine der Kammern in Fludiverbindung mit einem Einlaß und einem Auslaß steht sowie einer Druckwandlereinrichtung, die an der elektrisch isolierenden Zwischenwand derart angeordnet ist, daß sie deren Druck in elektrische Signale umwandelt.

Die DE 3207044 A1 betrifft ein Gerät zur invasiven Blutdruckmessung, bei dem ein elektromechanischer Wandler über umschaltbare Fluidventile mit Schlauchleitungen verbindbar ist, von denen eine Schlauchleitung sich zur Druckmessung an einen Druckmeßbereich erstreckt.

Aus der US 4,576,181 A ist ein weiterer Einmal-Transducer für die invasive Blutdruckmessung bekannt.

Die US-A-5 335 551 bezieht sich auf einen Kissentyp-Druckdetektor. Dieser Druckdetektor weist eine Trägervorrichtung, auf der ein Belastungssensor angebracht ist, eine bezüglich der Trägervorrichtung schwenkbare Abdeckvorrichtung, eine Lastübertragungsplatte sowie ein Druckreaktionsbauglied in Form eines beutelartigen Kissens auf. Das hohle Kissen ist mit einer Einlaßleitung und einer Auslaßleitung versehen. Bei dem in der US-A-5 335 551 offenbarten Detektor sind im Betrieb die Trägervorrichtung, der Belastungssensor, die Lastübertragungsplatte, der Beutel und die Abdeckvorrichtung derart angeordnet, daß durch einen in dem Beutel herrschenden Druck eine Kraft auf die Belastungsübertragungsplatte ausgeübt wird, die dann durch den Belastungssensor erfaßt wird. Der Beutel besteht dabei aus einem elastischen Material, beispielsweise Vinyl-Chlorid.

Ausgehend von diesem Stand der Technik liegt der Erfindung die Aufgabe zugrunde, eine Druckmeßvorrichtung zur invasiven Blutdruckmessung anzugeben, die zu einem erheblichen Teil mehrfach verwendet werden kann und eine gegenüber üblichen wiederverwendbaren Druckmeßvorrichtungen verbesserte Meßgenauigkeit aufweist.

Diese Aufgabe wird durch eine Druckmeßvorrichtung gemäß Patentanspuch 1 und eine Druckmeßvorrichtung gemäß Patentanspruch 8 gelöst.

Bei der erfindungsgemäßen Druckmeßvorrichtung gemäß dem ersten Aspekt der vorliegenden Erfindung wird die Trennung der potentiell infektiösen Flüssigkeit, in der der Druck gemessen wird, von dem wiederverwendbaren Rest der Druckmeßvorrichtung durch einen Schlauchkörper erreicht, der in Anlage gegen die Druckaufnahmefläche der Druckübertragungseinrichtung zwischen den Gehäuseteilen gehalten wird. Mit anderen Worten legt das zweite Gehäuseteil den Schlauch gegen die Druckaufnahmefläche innerhalb des ersten Gehäuseteils an, wodurch gewährleistet wird, daß die Druckübertragung weitgehend unabhängig von der Compliance des Schlauchkörpers, also der Volumendehnbarkeit des Schlauchkörpers ist.

Bei der erfindungsgemäßen Druckmeßvorrichtung gemäß dem zweiten Aspekt der vorliegenden Erfindung wird die Trennung der potentiell infektiösen Flüssigkeit, in der der Druck gemessen wird, von dem wiederverwendbaren Rest der Druckmeßvorrichtung durch einen Schlauchkörper erreicht, der in Anlage gegen die Druckaufnahmefläche gehalten wird. Vorzugsweise wird der Schlauch durch die Ausgestaltung des Schlauchhalterungsbereiches gegen die Druckaufnahmefläche gehalten, wodurch gewährleistet wird, daß die Druckübertragung weitgehend unabhängig von der Compliance des Schlauchkörpers, also der Volumendehnbarkeit des Schlauchkörpers ist. Dazu ist der Schlauchhalterungsbereich vorzugsweise in der Form eines Hohlraums ausgebildet, der den Schlauch mit Ausnahme der Einführungsöffnung im wesentlichen umgibt.

Bei der erfindungsgemäßen Druckmeßvorrichtung gemäß dem ersten und dem zweiten Aspekt der vorliegenden Erfindung sind alle aufwendigen Komponenten mehrfach verwendbar. Lediglich der Schlauchkörper sowie gegebenenfalls ein mit dem Schlauchkörper verbundenes Ventil zu seiner Entlüftung und ein Spülsystem sind Einwegkomponenten, wodurch die Belastung der Umwelt aufgrund des minimierten Müllaufkommens vermindert wird. Bei dem erfindungsgemäßen System gemäß dem ersten und dem zweiten Aspekt der vorliegenden Erfindung umfassen die Einwegbestandteile keinerlei elektronische Komponenten, so daß der Anfall von Elektronikschrott vollkommen vermieden wird.

Die erfindungsgemäße Druckmeßvorrichtung gemäß dem ersten und zweiten Aspekt der vorliegenden Erfindung ist gegenüber bekannten Mehrwegsystemen leichter mit Flüssigkeit befüllbar und entlüftbar. Die Handhabung des Gesamtsystems ist ausgesprochen einfach, mit nur geringem Erklärungsbedarf für das Klinikpersonal verbunden, da das Klinikpersonal daran gewöhnt ist, innerhalb ähnlicher Systeme die Entlüftung einfacher Schlauchkörper vorzunehmen. Weitere technische Handhabungen werden dem Klinikpersonal nicht abverlangt.

Bevorzugte Ausführungsbeispiele der vorliegenden Erfindung werden nachfolgend unter Bezugnahme auf die beiliegenden Zeichnungen näher erläutert. Es zeigen:
- Fig. 1: eine perspektivische Ansicht einer ersten Ausgestaltung der erfindungsgemäßen Druckmeßvorrichtung gemäß der ersten Aspekt der vorliegenden Erfindung;
- Fig. 2: eine Querschnittsdarstellung einer im wesentlichen mit der Ausführungsform von Fig. 1 übereinstimmenden zweiten Ausführungsform einer erfindungsgemäßen Druckmeßvorrichtung gemäß dem ersten Aspekt der vorliegenden Erfindung ;
- Fig. 3 bis 6: mögliche Ausgestaltungen der bei der erfindungsgemäßen Druckmeßvorrichtung verwendbaren Schlauchkörper;
- Fig. 7: eine perspektivische Ansicht einer zweiten Ausgestaltung der erfindungsgemäßen Druckmeßvorrichtung gemäß dem ersten Aspekt der vorliegenden Erfindung;
- Fig. 8: eine Querschnittdarstellung eines ersten Ausführungsbeispiels der erfindungsgemäßen Druckmeßvorrichtung gemäß dem zweiten Aspekt der vorliegenden Erfindung; und
- Fig. 9: eine perspektivische Ansicht eines weiteren Ausführungsbeispiels der erfindungsgemäßen Druckmeßvorrichtung gemäß dem zweiten Aspekt der vorliegenden Erfindung.

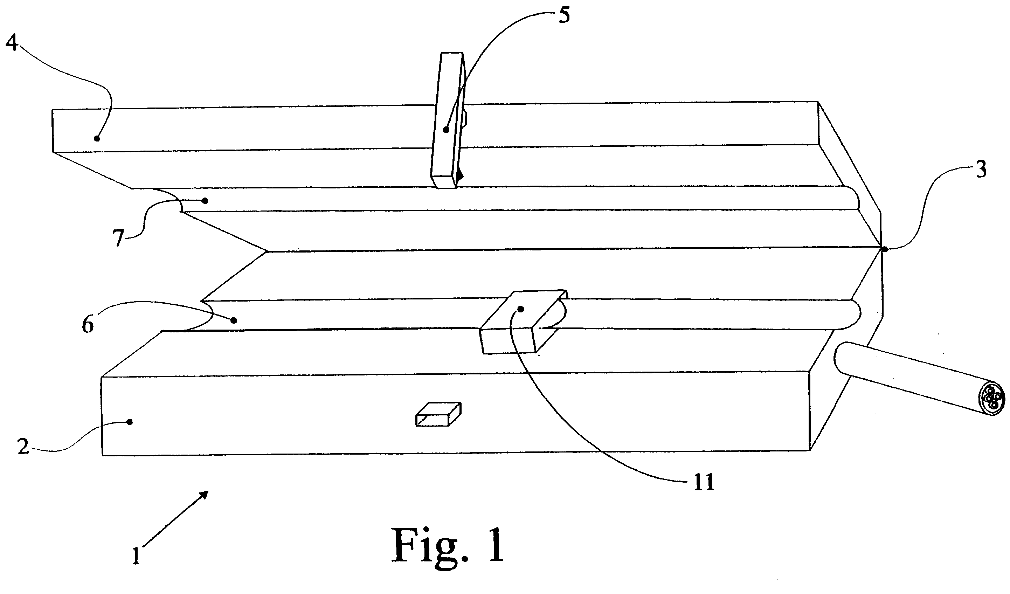

In Fig. 1 ist eine Blutdruckmeßvorrichtung für die invasive Blutdruckmessung gemäß dem ersten Aspekt der vorliegenden Erfindung in ihrer Gesamtheit mit dem Bezugszeichen 1 bezeichnet und umfaßt ein erstes Gehäuseteil 2, an dem über ein oder mehrere Scharniere 3 ein zweites Gehäuseteil schwenkbeweglich befestigt ist. Das zweite Gehäuseteil 4 ist in seiner gegen das erste Gehäuseteil 2 angeklappten Schwenklage an diesem durch einen Verschlußmechanismus 5 oder mehrere Verschlußmechanismen verrastbar. Die beiden Gehäuseteile 2, 4 weisen je eine sich über deren Länge erstreckende Schlauchführungsausnehmung 6, 7 mit jeweils halbkreisförmigem Querschnitt auf. Anmerkung: Der Querschnitt muß nicht unbedingt kreisförmig sein.

Das erste Gehäuseteil 2 weist einen (in Fig. 1 nicht sichtbaren) Drucksensor 8 auf, der über eine sich in den ersten Gehäuseteil 2 erstreckende Druckübertragungseinrichtung 9, 10 mit einer Druckaufnahmefläche 11 in Druckübertragungsverbindung steht.

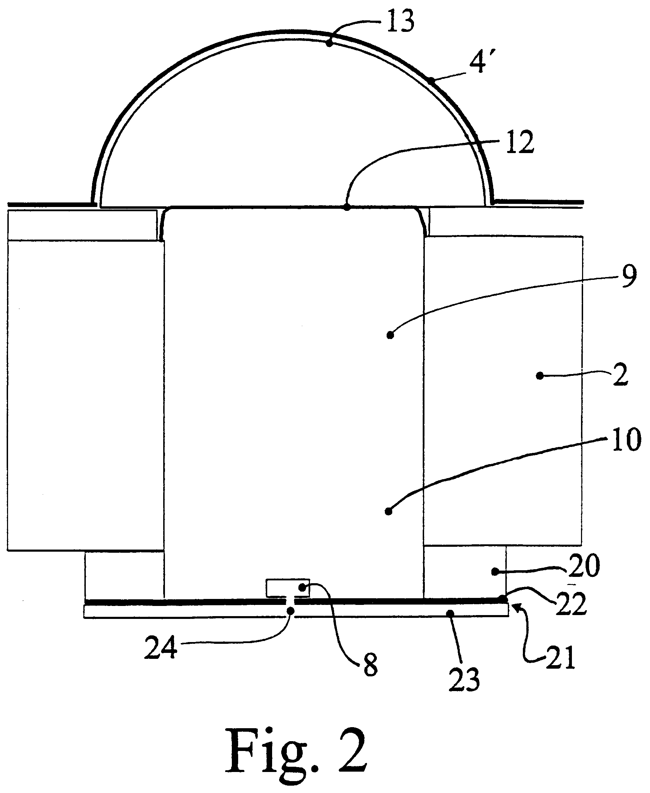

Wie dies bei dem in Fig. 2 gezeigten Ausführungsbeispiel verdeutlicht ist, kann die Druckübertragungseinrichtung beispielsweise durch eine Silikongelfüllung 9 eines Hohlraumes 10 in dem ersten Gehäuseteil 2 gebildet sein, wobei die Silikongelfüllung 9 in Richtung auf das zweite Gehäuseteil 4', welches bei der in Fig. 2 gezeigten Ausführungsform als halbrunder Gehäusedeckel ausgeführt ist, durch eine Schutzfolie 12 abgegrenzt ist, welche die Druckaufnahmefläche 11 festlegt.

Wie insbesondere in Fig. 2 verdeutlicht ist, umfaßt die Blutdruckmeßvorrichtung einen Schlauchkörper 13, an dessen Querschnitt die Schlauchführungsausnehmung 6, 7 in den beiden Gehäuseteilen 2, 4 angepaßt sind. Das zweite Gehäuseteil 4, 4' drückt den Schlauchkörper 13 gegen die Druckaufnahmefläche 11, welche bei dem Ausführungsbeispiel der Fig. 2 durch die Schutzfolie 12 oberhalb der Silikongelfüllung 9 gebildet ist.

An den Boden des ersten Gehäuseteils 2 grenzt eine Dichtung 20 an. Eine Bodenplatte 21 ist mittels der Dichtung 20 an der Unterseite des ersten Gehäuseteils 2 dichtend festgelegt. Die Bodenplatte 21 umfaßt eine Keramikschicht 22, die als Träger des Drucksensors 8 dient, sowie eine Abschirmung 23. Die Bodenplatte 21 weist im Bereich des Drucksensors 8 eine Entlüftungsöffnung 24 auf, mit der gewährleistet wird, daß der auf den Drucksensor 8 wirkende Druck gegenüber dem Atmosphärendruck gemessen wird.

In Abweichung von dem in Fig. 2 gezeigten Ausführungsbeispiel kann die Druckübertragungseinrichtung auch durch eine elastische Membran gebildet sein, die das erste Gehäuseteil 1 in Richtung zum zweiten Gehäuseteil 4 abgrenzt und einen Drucksensor überspannt. Jegliche Einrichtung, die den von dem Schlauchkörper auf die Druckaufnahmefläche ausgeübten Druck auf den Drucksensor überträgt, kann als Druckübertragungseinrichtung im Sinne der Terminologie der vorliegenden Anmeldung verwendet werden.

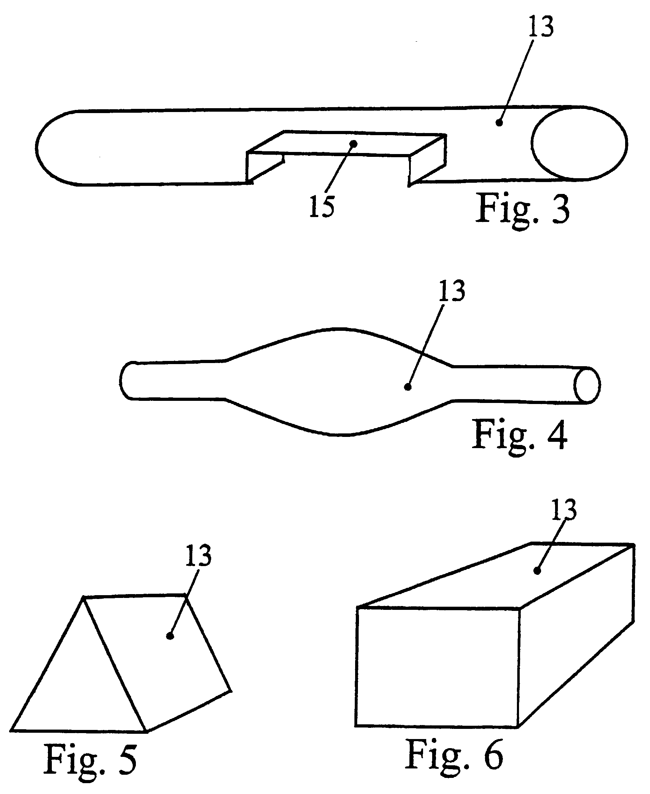

Der Schlauchkörper kann unterschiedlichste Ausgestaltungen haben, von denen nur einige schematisch in den Fig. 3 bis 6 dargestellt sind. In der einfachsten Ausführungsform ist der Schlauchkörper ein Schlauch mit kreisförmigem Querschnitt, welcher als Folienschlauch implementiert sein kann. Hinsichtlich Beständigkeit, der erforderlichen Verklebungstechnik und der Verträglichkeit beim Menschen erwiesen sich nach Versuchen Silikonschläuche als geeignet. Bei einem bevorzugten Auführungsbeispiel umfassen die Silikonschläuche an ihrem distalen Ende ein Absperrventil, das zur Entlüftung des Schlauches, also einer Flüssigkeitsbefüllung dient (nicht dargestellt) und am anderen (proximalen) Ende ein handelsübliches Spülsystem, das aus einer Druckinfusion einen konstanten Spülfluß erlaubt und durch Betätigung eines Mechanismus einen erhöhten Fluß bereitstellt. Bevorzugt erstreckt sich der Schlauchkörper einstückig bis zu einem Katheter, der das Drucksignal aufnimmt. Es ist jedoch auch möglich, den Schlauchkörper an einen weiteren Schlauch anzuschließen, der seinerseits mit dem Katheter verbunden ist.

Es kommen runde, im Querschnitt dreieckige, viereckige oder mehreckige und ovale Schlauchformen ebenso wie unregelmäßige Querschnittsausgestaltungen des Schlauchkörpers in Betracht.

Bei der in Fig. 3 gezeigten Ausführungsform ist derjenige Schlauchbereich, der zur Anlage an die Druckaufnahmefläche 11 bestimmt ist, abgeflacht ausgestaltet.

Ebenfalls ist es möglich, einen in dem Anlagebereich der Druckaufnahmefläche 11 aufgetriebenen Schlauchkörper für die Zwecke der Erfindung zu verwenden, wie dieser in Fig. 4 dargestellt ist.

Die Fig. 5 und 6 zeigen Ausführungsbeispiele der erwähnten, im Querschnitt dreieckigen und viereckigen Schlauchkörper. Die Form der Schlauchkörper ist, wie erwähnt, nicht auf die gezeigten Ausgestaltungen beschränkt.

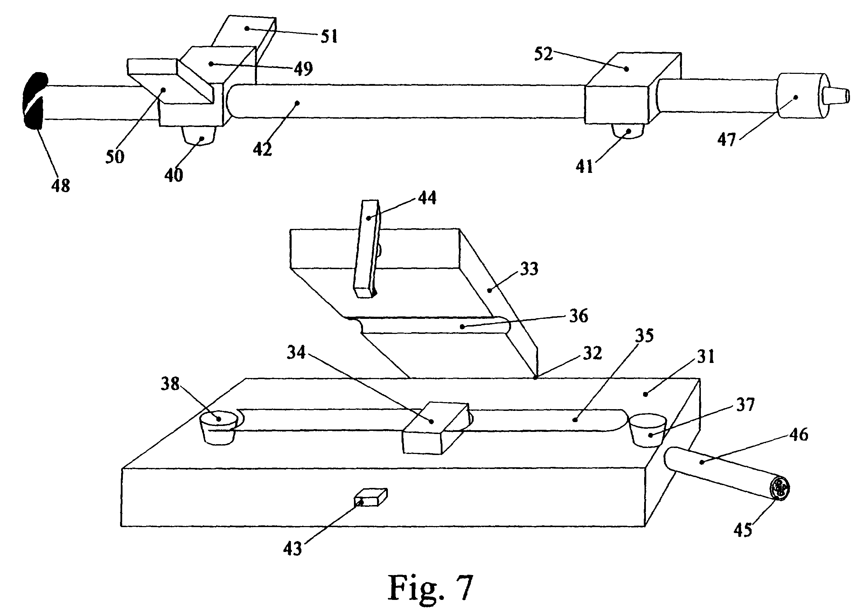

Fig. 7 zeigt eine zweite Ausführungsform der erfindungsgemäßen Blutdruckmeßvorrichtung gemäß dem ersten Aspekt der vorliegenden Erfindung, die wiederum ein erstes Gehäuseteil 31 aufweist, an dem über ein Scharnier 32 ein zweites Gehäuseteil 33 schwenkbeweglich festgelegt ist. An dem mit dem Bezugszeichen 34 verdeutlichten Druckaufnahmebereich liegt wiederum ein Drucksensor 8 mit einer Druckübertragungseinrichtung, welche die in Fig. 2 gezeigte und unter Bezugnahme auf Fig. 2 näher erläuterte Struktur haben kann.

In den beiden Gehäuseteilen 31, 33 sind Schlauchführungsausnehmungen 35, 36 vorgesehen. Im ersten Gehäuseteil 31 sind ferner zwei Einrastausnehmungen 37, 38 angeordnet, welche zur Festlegung von Rastnasen 40, 41 zur Festlegung eines Schlauchkörpers 42 gegenüber den Gehäuseteilen 31, 33 dienen. Das erste Gehäuseteil 31 hat ferner eine Rastkerbe 43, die mit einem an dem zweiten Gehäuseteil 33 festgelegten Verschlußmechnismus 44 in Eingriff bringbar ist. Zum Anschluß des in dem ersten Gehäuseteil 31 angeordneten Drucksensors (nicht dargestellt) dient ein mit einer Abschirmung 45 versehenes Kabel 46.

Der Schlauchkörper 42 ist an seinem einen Ende mit einem männlichen Luer-Verbinder 47 und an seinem anderen Ende mit einem weiblichen Luer-Verbinder 48 versehen. Ein Spülsystemkörper 49 mit Spülkanälen schließt an den Schlauchkörper 42 an und trägt die erste Rastnase 40. Flügel 50, 51 dienen zur manuellen Betätigung des Spülkanals zum Freispülen des Katheters. Ein Schlauchhalterungskörper 52 umschließt den Schlauchkörper 42 an einer von dem Spülsystemkörper 49 longitudinal beabstandeten Stelle und dient zur Festlegung der zweitgenannten Rastnase 41.

Wie bereits erwähnt, weist das Kabel 46 eine Abschirmung 45 auf. Diese Abschirmung 45 ist einerseits an den Masseanschluß eines Auswertungsmonitors angeschlossen. Andererseits steht sie mit der Abschirmung 23 der Bodenplatte 21 (vergleiche Fig. 2) in Verbindung. Hierdurch werden elektrostatische Störungen der Messung verhindert.

Bei den bislang beschriebenen Ausführungsbeipielen sind die beiden Gehäuseteile miteinander schwenkbeweglich über ein Scharnier verbunden. Es ist jedoch möglich, die gegenseitige Festlegung der Gehäuseteile mit jeglichen anderen lösbaren Verbindungsmitteln, wie beispielsweise Rastverbindungen oder Schraubverbindungen, vorzunehmen.

In Abweichung von den gezeigten Ausführungsbeispielen kann der Schlauchkörper fest mit dem zweiten Gehäuseteil verbunden sein, welches dann zur Festlegung des Schlauchkörpers an dem ersten Gehäuseteil rastbar festgelegt werden kann. In diesem Fall ist das zweite Gehäuseteil gleichzeitig mit dem Schlauchkörper bei jeder Messung zu ersetzen.

Bei der unter Bezugnahme auf Fig. 7 beschriebenen Ausführungsform ist die Festlegung der Anordnung des Schlauchkörpers durch Rastnasen und entsprechende Einrastausnehmungen gegeben. Die gegenseitige Festlegung kann jedoch auch durch eine angepaßte Außenkontur des Schlauchkörpers an die Gestaltung der Gehäuseteile erfolgen. So kann beispielsweise der Schlauchkörper eine runde oder ellipsoide Aushöhlung aufweisen, die mit einer entsprechenden Gegenform in einem Gehäuseteil eine formflüssige Festlegung der Lage des Schlauchkörpers bewirkt.

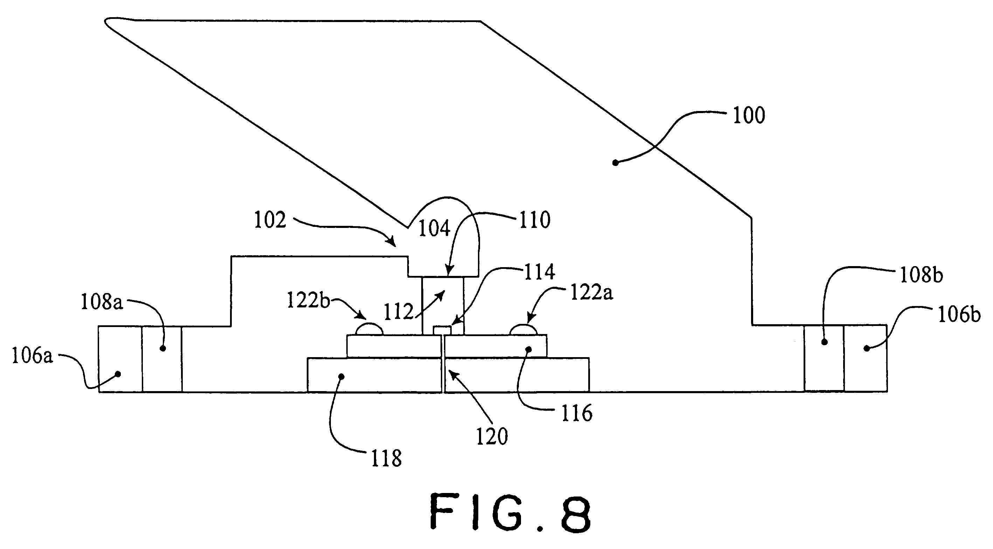

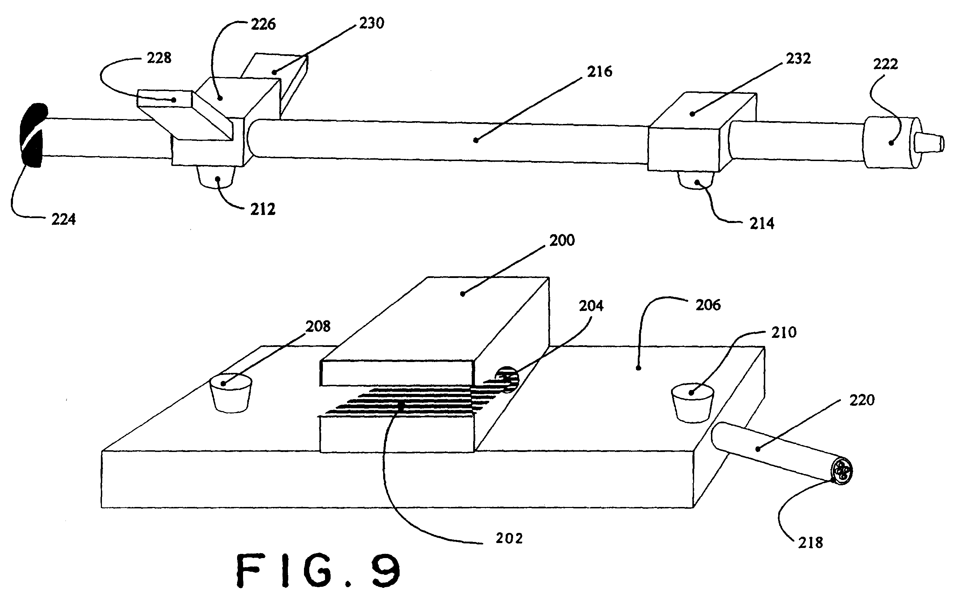

Die erfindungsgemäße Blutdruckmeßvorrichtung gemäß dem zweiten Aspekt der vorliegenden Erfindung wird nachfolgend anhand der Fig. 8 und 9 näher erläutert.

Die Blutdruckmeßvorrichtung weist ein Gehäusebauteil 100 auf. Der Gehäusebauteil kann einen quaderförmigen Umriß aufweisen, wie beispielsweise in Fig. 9 dargestellt ist, oder kann einen Querschnitt aufweisen, wie er in Fig. 8 dargestellt ist. In dem Gehäusebauteil ist eine Einführungsöffnung 102 vorgesehen. Durch diese Einführungsöffnung 102 kann ein Schlauchkörper, der in Fig. 8 nicht dargestellt ist, in einen Schlauchhalterungsbereich 104 in dem Gehäusebauteil 100 eingeführt werden. Der Schlauchhalterungsbereich ist bei dem bevorzugten Ausführungsbeispiel der vorliegenden Erfindung durch einen Hohlraum in dem Gehäusebauteil 100 gebildet. Wie ferner in Fig. 8 zu sehen ist, ist die Einführungsöffnung 102 ausgehend von der inneren, den Hohlraum, der den Schlauchhalterungsbereich 104 definiert, begrenzenden Wand zur Außenseite des Gehäusebauteils 100 hin aufgeweitet, um ein leichteres Einführen eines Schlauchkörpers in den Schlauchhalterungsbereich zu ermöglichen. Wie ebenfalls in Fig. 8 dargestellt ist, kann das Gehäusebauteil 100 seitliche Verlängerungen 106a, 106b aufweisen, in denen Ausnehmungen 108a, 108b vorgesehen sind, um das Gehäusebauteil an einer weiteren Vorrichtung zu befestigen.

Der in Fig. 8 dargestellte Schlauchhalterungsbereich 104 dient zur Aufnahme eines Schlauchkörpers mit einem kreisförmigen Querschnitt. Angrenzend an den Schlauchhalterungsbereich 104, d.h. bei dem bevorzugten Ausführungsbeispiel an den in dem Gehäusebauteil gebildeten Hohlraum, ist eine Druckaufnahmefläche 110 angeordnet. Die Druckaufnahmefläche 110 steht über eine Druckübertragungeinrichtung 112 mit einem Drucksensor 114 in Verbindung.

Die Druckübertragungseinrichtung 112 kann beispielsweise durch eine Silikongelfüllung eines Hohlraums in dem Gehäusebauteil 100 gebildet sein, wobei die Silikongelfüllung zu dem Hohlraum, der den Schlauchhalterungsbereich 104 definiert, hin durch eine Schutzfolie abgegrenzt ist, welche die Druckaufnahmefläche 110 festlegt. Das Gehäuseteil 100 kann über der Druckaufnahmeeinrichtung eine wiederverschließbare Bohrung aufweisen, durch die das Silikongel in den Raum der Druckübertragungseinrichtung 112 befüllt werden kann.

Das Gehäusebauteil 100 weist eine geeignete Ausnehmung auf, in der eine Bodenplatte 116, auf der der Drucksensor 114 angeordnet ist, angeordnet ist. Die Bodenplatte 116 kann beispielsweise eine Keramikschicht umfassen, die als Träger für den Drucksensor 114 dient. Unter der Bodenplatte 116 kann eine elektrisch leitende Abschirmung angebracht sein, die in der Zeichnung nicht dargestellt ist. Ein Deckel 118 hält die Bodenplatte 116 in Anlage an das Gehäuseteil 100. Die Bodenplatte 116 und der Deckel 118 weisen im Bereich des Drucksensors 114 vorzugsweise eine Entlüftungsöffnung 120 auf, mit der gewährleistet wird, daß der auf den Drucksensor 114 wirkende Druck gegenüber dem Atmosphärendruck gemessen wird.

Die Bodenplatte 116 ist abdichtend in der Ausnehmung des Gehäusebauteils 100 angebracht. Vorrichtungen zum Befestigen der Bodenplatte an dem Gehäusebauteil 100 sind schematisch bei 122a und 122b dargestellt.

Alternativ zu dem oben beschriebenen Beispiel kann die Druckübertragungseinrichtung auch durch eine elastische Membran gebildet sein, die einen Drucksensor überspannt und eine Abgrenzung von dem Drucksensor zu dem den Schlauchhalterungsbereich definierenden Hohlraum darstellt. Jegliche Einrichtung, die den von dem Schlauchkörper auf die Druckaufnahmefläche ausgeübten Druck auf den Drucksensor überträgt, kann als Druckübertragungseinrichtung im Sinne der Terminologie der vorliegenden Anmeldung verwendet werden.

Im Betrieb wird ein Schlauchkörper, der in Fig. 8 nicht dargestellt ist, dessen Längsrichtung in die in Fig. 8 gezeigte Zeichenebene verläuft, durch die Einführungsöffnung 102 in den Schlauchhalterungsbereich 104 eingeführt. Dabei ist der Querschnitt der Einführungsöffnung 102 vorzugsweise etwas kleiner als der Durchmesser des Schlauchkörpers, so daß zum Einführen des Schlauchkörpers ein leichtes Zusammendrücken des Schlauchkörpers notwendig ist. Wenn der Schlauchkörper in den Hohlraum, der den Schlauchhalterungsbereich 104 definiert, eingebracht ist, ist er in Anlage gegen die Druckaufnahmefläche 110 gehalten. Bei der in Fig. 8 dargestellten Ausführungsform des Schlauchhalterungsbereichs 104 ist der Bereich desselben, in dem die Druckaufnahmefläche 110 angeordnet ist, flach. Die restliche innere Wandung des Hohlraums, der den Schlauchhalterungsbereich definiert, entspricht im wesentlichen dem Umfang des Schlauchkörpers, wodurch die Anlage einer Wand des Schlauchkörpers gegen die Druckaufnahmefläche 110 gewährleistet ist.

Fig. 9 ist eine grobe schematische Darstellung einer Druckmeßvorrichtung gemäß dem zweiten Aspekt der vorliegenden Erfindung. Ein Gehäusebauteil 200 weist eine Einführungsöffnung 202 und einen Schlauchhalterungsbereich 204 auf. Wie in Fig. 9 zu sehen ist, erstreckt sich der Schlauchhalterungsbereich 204 entlang der Breite des Gehäusebauteils 200 und ist an beiden Seiten offen, derart, daß ein Schlauch durch die Einführungsöffnung 202, die sich ebenfalls über die Breite des Gehäusebauteils 200 erstreckt, in den Schlauchhalterungsbereich 204 eingeführt werden kann. Der Schlauch verläuft dann quer durch das Gehäusebauteil 200, wobei er dasselbe auf einer Seite betritt und auf der anderen Seite verläßt. Die zur Druckerfassung notwendigen Elemente des Gehäusebauteils 200 sind in Fig. 9 nicht dargestellt.

Bei dem in Fig. 9 dargestellten Ausführungsbeispiel ist das Gehäusebauteil 200 auf einer Trägerplatte 206 angeordnet. Alternativ kann die Trägerplatte 206 ein Bestandteil des Gehäusebauteils 200 sein, wobei das Gehäusebauteil 200 und die Trägerplatte 206 einstückig ausgebildet sind. In der Trägerplatte 206 sind zwei Einrastausnehmungen 208 und 210, die beispielsweise durch Nuten, Aussparungen oder Einkerbungen gebildet sein können, angeordnet. Die Einrastausnehmungen 208 und 210 dienen zur Festlegung von Rastnasen 212, 214 zur Festlegung eines Schlauchkörpers 216 gegenüber dem Träger 206 und somit dem Gehäusebauteil 200. Zum Anschluß des in dem Gehäusebauteil 200 angeordneten Drucksensors (nicht dargestellt) dient ein mit einer Abschirmung 218 versehenes Kabel 220.

Der Schlauchkörper 216 ist an seinem einen Ende vorzugsweise mit einem männlichen Luer-Verbinder 222 und seinem anderen Ende mit einem weiblichen Luer-Verbinder 224 versehen. Ein Spülsystemkörper 226 mit Spülkanälen schließt an den Schlauchkörper 216 an und trägt die erste Rastnase 212. Flügel 228, 230 dienen zur manuellen Betätigung des Spülkanals zum Freispülen des Katheters. Ein Schlauchhalterungskörper 232 umschließt den Schlauchkörper 216 an einer von dem Spülsystemkörper 246 longitudinal beabstandeten Stelle und dient zur Festlegung der zweiten Rastnase 214.

Zwischen dem Spülsystemkörper 226 und dem Schlauchhalterungskörper kann eine Verbindungseinrichtung (in Fig. 9 nicht dargestellt) implementiert sein, die den Schlauchkörper überbrückt. Diese Verbindungseinrichtung kann derart ausgestaltet sein, daß sie die Einführungsöffnung 202 ausfüllt, wobei dieselbe vorzugsweise an den Querschnitt der Einführungsöffnung angepaßt ist. Dadurch kann verhindert werden, daß sich der Schlauchkörper 216 aus dem Schlauchhalterungsbereich 204 in die Einführungsöffnung 202 herauswölbt. Die Verbindungseinrichtung kann ferner das Einführen des Schlauchkörpers 216 durch die Einfünrungsöffnung in den Schlauchhalterungsbereich erleichtern. Beim Vorliegen der Verbindungseinrichtung können statt der bezugnehmend auf Fig. 9 beschriebenen Einrastausnehmungen 208 und 210 entsprechende Haltemechanismen für die Verbindungseinrichtung beispielsweise seitlich an dem Gehäusebauteil 200 angeordnet sein.

Wie bereits erwähnt wurde, weist das Kabel 220 eine Abschirmung 218 auf. Diese Abschirmung ist einerseits an den Masseanschluß eines Auswertungsmonitors angeschlossen. Andererseits steht sie mit der Abschirmung der Bodenplatte 116 (siehe Fig. 8) in Verbindung. Hierdurch werden elektrostatische Störungen der Messung verhindert. Bei der bezugnehmend auf Fig. 9 beschriebenen Ausführungsform ist die Festlegung der Anordnung des Schlauchkörpers durch Rastnasen und entsprechende Einrastausnehmungen gegeben. Die gegenseitige Festlegung kann jedoch auch durch eine angepaßte Außenkontur des Schlauchkörpers an die Gestaltung des Schlauchhalterungsbereichs erfolgen. Der Schlauchkörper kann beispielsweise eine runde oder ellipsoide Aushöhlung aufweisen, die mit einer entsprechenden Gegenform entweder innerhalb des Schlauchhalterungsbereichs oder im Bereich der Trägerplatte 206 eine formschlüssige Festlegung der Lage des Schlauchkörpers bewirkt.

Bei der in Fig. 3 gezeigten Ausführungsform ist ein Schlauchbereich 15 des Schlauchkörpers, der zur Anlage an die Druckaufnahmefläche 110 bestimmt ist, abgeflacht ausgestaltet. Dies kann vorteilhaft sein, wenn die Druckaufnahmefläche 110 in dem Schlauchhalterungsbereich leicht erhöht ist. Ferner kann durch eine derartige Ausgestaltung eine Festlegung des Schlauchkörpers gegenüber dem Gehäusebauteil 100 erfolgen.

Es ist ferner offensichtlich, daß die Form des Schlauchhalterungsbereichs vorzugsweise jeweils an die Form des verwendeten Schlauchkörpers angepaßt ist, um eine sichere Anlage des Schlauchs gegen die Druckaufnahmefläche 110 zu gewährleisten.

Neben den speziell beschriebenen Ausführungsbeispielen umfaßt die vorliegende Erfindung eine Vielzahl von Alternativen zu denselben. Beispielsweise könnte die Einführungsöffnung oben oder unten im Gehäusebauteil angeordnet sein. Ferner können der Sensor und die Übertragungseinrichtung oberhalb des Schlauchkörpers zu liegen kommen, so daß der gesamte Sensor quasi in einer Kopfüber-Stellung angeordnet ist. Damit ist das System besser gegen Spritzwasser geschützt.

Alternativ zu den beschriebenen Ausführungsbeispielen kann die erfindungsgemäße Druckmeßvorrichtung ferner einen solchen Aufbau aufweisen, daß der komplette Druckaufnehmer mit Druckübertragungseinrichtung, Bodenplatte, usw., d.h. die eigentliche Druckmeßvorrichtung, in eine entsprechende Ausnehmung des Gehäusebauteils eingerastet ist. Dadurch ist die eigentliche Druckmeßvorrichtung von dem Gehäusebauteil trennbar. Dies ermöglicht eine getrennte Entsorgung von Druckmeßvorrichtung und Gehäusebauteil, bzw. den einzelnen Austausch einer der beiden Komponenten.

## Patentansprüche

1. Druckmeßvorrichtung zur invasiven Blutdruckmessung mit folgenden Merkmalen:
einem ersten Gehäuseteil (2);
einem an dem ersten Gehäuseteil (2) angeordneten Drucksensor (8);
einer sich zwischen dem Drucksensor (8) und einer Druckaufnahmefläche (11) in dem ersten Gehäuseteil (2) erstreckenden Druckübertragungseinrichtung (9, 10, 12);
einem mit einer Flüssigkeit zur Druckübertragung von einem Druckmeßbereich befüllten Schlauchkörper (13); und
einem gegenüber dem ersten Gehäuseteil (2) festlegbaren zweiten Gehäuseteil (4), das den Schlauchkörper (13) für eine Druckübertragung in Anlage gegen die Druckaufnahmefläche (11) hält,
dadurch gekennzeichnet, daß
das erste Gehäuseteil (2) eine erste Schlauchführungsausnehmung (6) aufweist, die sich beidseitig von der Druckaufnahmefläche (11) aus erstreckt, wobei das zweite Gehäuseteil (4) eine zweite Schlauchführungsausnehmung (7) aufweist, und wobei die erste und die zweite Schlauchführungsausnehmung den Schlauch im Bereich der Druckaufnahmefläche im wesentlichen umgeben, wenn das zweite Gehäuseteil (4) gegenüber dem ersten Gehäuseteil festgelegt ist, derart, daß die Druckübertragung weitgehend unabhängig von der Volumendehnbarkeit des Schlauchkörpers (13) ist.

2. Druckmeßvorrichtung nach Anspruch 1, bei der der zweite Gehäuseteil (4) an dem ersten Gehäuseteil (2) durch einen oder mehrere Scharniere (3) befestigt ist.

3. Druckmeßvorrichtung nach Anspruch 2, bei der der zweite Gehäuseteil (4) in einer gegen das erste Gehäuseteil (2) um das Scharnier (3) angeklappten Lage durch einen Verschlußmechanismus (5) gehalten ist.

4. Druckmeßvorrichtung nach einem der Ansprüche 1 bis 3, bei der die Druckübertragungseinrichtung durch einen mit einem Gel (9) gefüllten Hohlraum (10) des ersten Gehäuseteils (2) gebildet ist, der sich von der Druckaufnahmefläche (11) bis zu dem Drucksensor (8) erstreckt.

5. Druckmeßvorrichtung nach Anspruch 4, bei der der mit Gel (9) befüllte Hohlraum (10) im Bereich der Druckaufnahmefläche (11) mit einer Schutzfolie (12) überzogen ist.

6. Druckmeßvorrichtung nach einem der Ansprüche 1 bis 5, bei der das erste Gehäuseteil (31) zwei Einrastausnehmungen (37,38) aufweist, in die Rastnasen (40,41) einführbar sind, welche mit dem Schlauchkörper (42) verbunden sind.

7. Druckmeßvorrichtung nach einem der Ansprüche 1 bis 6, bei der das erste Gehäuseteil (31) eine Luer-kompatible Bohrung (24) aufweist, über die eine angrenzende Seite des Drucksensors (8) mit der Atmosphäre in Verbindung steht.

8. Druckmeßvorrichtung zur invasiven Blutdruckmessung mit folgenden Merkmalen:
einem Gehäusebauteil (100; 200);
einem an dem Gehäusebauteil (100; 200) angeordneten Drucksensor (114);
einer sich zwischen dem Drucksensor (114) und einer Druckaufnahmefläche (110) in dem Gehäusebauteil (100; 200) erstreckenden Druckübertragungseinrichtung (112); und
einem mit einer Flüssigkeit zur Druckübertragung von einem Druckmeßbereich befüllten Schlauchkörper (216);
dadurch gekennzeichnet, daß
das Gehäusebauteil (100; 200) eine Einführungsöffnung (102; 202) und einen Schlauchhalterungsbereich (104; 204) aufweist, wobei der Schlauchkörper (216) durch die Einführungsöffnung (102; 202) in den Schlauchhalterungsbereich (104; 204) einbringbar ist und mit einer Wand des Schlauchkörpers (216) in Anlage gegen die Druckaufnahmefläche (110) gehalten ist, wenn er in den Schlauchhalterungsbereich (104; 204) eingebracht ist, und
der Schlauchhalterungsbereich (104; 204) als länglicher Hohlraum in dem Gehäusebauteil (100; 200) ausgebildet ist, wobei in einem Abschnitt der den Hohlraum umgebenden länglichen Seitenwandung des Hohlraums die Einführungsöffnung (102; 202) angeordnet ist, wobei die Wand des Schlauchkörpers (216) durch die längliche Seitenwandung des Hohlraums, die den Schlauchkörper (216) im Bereich der Druckaufnahmefläche (110) im wesentlichen umgibt, in Anlage gegen die Druckaufnahmefläche (110) gehalten ist, wenn der Schlauchkörper in den Hohlraum eingebracht ist, derart, daß die Druckübertragung weitgehend unabhängig von der Volumendehnbarkeit des Schlauchkörpers (216) ist.

9. Druckmeßvorrichtung gemäß Anspruch 8, bei der die Einführungsöffnung (102; 202) eine längliche Öffnung ist, durch die der Schlauchkörper (216) mittels einer Bewegung senkrecht zu seiner Längsausdehnung in den Schlauchhalterungsbereich (104; 204) einbringbar ist.

10. Druckmeßvorrichtung gemäß Anspruch 9, bei der der Schlauchkörper (216) aus einem flexiblen Material besteht, und bei der der Einführungsquerschnitt der Einführungsöffnung (102; 202) in der Richtung senkrecht zu der länglichen Ausdehnung kleiner ist als der Durchmesser des Schlauchkörpers (216).

11. Druckmeßvorrichtung gemäß Anspruch 10, bei der die Querschnittfläche des länglichen Hohlraums im wesentlichen der Querschnittfläche des Schlauchkörpers (216) entspricht.

12. Druckmeßvorrichtung gemäß Anspruch 10, bei der die längliche Seitenwandung des Hohlraums in dem Bereich, in dem die Druckaufnahmefläche (110) angeordnet ist, flach ist.

13. Druckmeßvorrichtung gemäß einem der Ansprüche 10 bis 12, bei der die Einführungsöffnung (102; 202) ausgehend von der inneren, den Hohlraum begrenzenden Wand des Gehäusebauteils (100; 200) durch die Wand des Gehäusebauteils (100; 200) nach außen aufgeweitet ist.

14. Druckmeßvorrichtung gemäß einem der Ansprüche 8 bis 13, bei der die Druckübertragungseinrichtung (112) durch einen mit einem Gel gefüllten Hohlraum des Gehäusebauteils (100; 200) gebildet ist, der sich von der Druckaufnahmefläche (110) bis zu dem Drucksensor (114) erstreckt.

15. Druckmeßvorrichtung gemäß einem der Ansprüche 8 bis 14, bei der das Gehäusebauteil (200, 206) zwei Einrastausnehmungen (208, 210) aufweist, in die Rastnasen (212, 214) einfügbar sind, welche mit dem Schlauchkörper (216) verbunden sind.

16. Druckmeßvorrichtung gemäß einem der Ansprüche 8 bis 15, bei der das Gehäusebauteil (100; 200) eine Luer-kompatible Bohrung (120) aufweist, über die eine angrenzende Seite des Drucksensors (114) mit der Atmosphäre in Verbindung steht.

17. Druckmeßvorrichtung gemäß einem der Ansprüche 8 bis 16, bei der an dem Schlauchkörper (216) eine Verbindungseinrichtung angeordnet ist, die die Einführungsöffnung (202) im wesentlichen verschließt, wenn der Schlauchkörper in den Schlauchhalterungsbereich (204) eingebracht ist.

18. Druckmeßvorrichtung nach einem der Ansprüche 1 bis 17, bei der der Schlauchkörper (13; 216) einen im wesentlichen runden Querschnitt hat.

19. Druckmeßvorrichtung nach einem der Ansprüche 1 bis 17, bei der der Schlauchkörper (13; 216) einen im wesentlichen ovalen Querschnitt hat.

20. Druckmeßvorrichtung nach einem der Ansprüche 1 bis 17, bei der der Schlauchkörper (13; 216) einen im wesentlichen mehreckigen Querschnitt hat.

21. Druckmeßvorrichtung nach einem der Ansprüche 1 bis 20, bei der der Schlauchkörper (13; 216) in seinem Anlagebereich an der Druckaufnahmefläche (11; 110) eine gegenüber seiner sonstigen Querschnittsfläche erhöhte Querschnittsfläche aufweist.

22. Druckmeßvorrichtung nach einem der Ansprüche 1 bis 20, bei der der Schlauchkörper (13; 216) in seinem Anlagebereich an der Druckaufnahmefläche (11; 110) einen ebenen Wandbereich (15) aufweist.

23. Druckmeßvorrichtung nach einem der Ansprüche 1 bis 22, bei der sich der Schlauchkörper (13; 216) einstückig bis zu einem offenen Schlauchende erstreckt, an dem dessen Flüssigkeitsfüllung bei der Blutdruckmessung mit dem Blutdruckmeßbereich in Druckverbindung steht.

24. Druckmeßvorrichtung nach Anspruch 23, bei der der Schlauchkörper (13; 216) an seinem von dem offenen Schlauchende entfernten distalen Ende mit einem Entlüftungsventil versehen ist.

25. Druckmeßvorrichtung nach einem der Ansprüche 1 bis 24, bei der ein Ende des Schlauchkörpers (42; 216) mit einem Spülsystemkörper (49; 226) versehen ist.

## Claims

1. A pressure-measuring device for invasive blood pressure measurement comprising:
a first housing component (2);
a pressure sensor (8) arranged on said first housing component (2);
a pressure-transmitting means (9, 10, 12) extending between said pressure sensor (8) and a pressure-taking surface (11) in said first housing component (2);
a hose body (13) filled with a fluid for transmitting pressure from a pressure-measuring area; and
a second housing component (4), which is adapted to be secured in position relative to the first housing component (2) and which holds the hose body (13) in contact with the pressure-taking surface (11) for pressure transmission,
characterized in that
the first housing component (2) is provided with a first hose-guiding recess (6) extending from the pressure-taking surface (11) on both sides thereof, said second housing component (4) being provided with a second hose-guiding recess (7), and said first and second hose-guiding recesses essentially enclosing the hose in the area of the pressure-taking surface, when the second housing component (4) is secured in position relative to the first housing component, in such a way that pressure transmission is largely independent of the volume expansibility of the hose body (13).

2. A pressure-measuring device according to claim 1, wherein the second housing component (4) is secured to the first housing component (2) by one or by a plurality of hinges (3).

3. A pressure-measuring device according to claim 2, wherein the second housing component (4) is held by a closure mechanism (5) after having been pivoted about the hinge (3) to a position of contact with the first housing component (2).

4. A pressure-measuring device according to one of the claims 1 to 3, wherein the pressure-transmitting means is defined by a cavity (10) of the first housing component (2), said cavity being filled with a gel (9) and extending from the pressure-taking surface (11) to the pressure sensor (8).

5. A pressure-measuring device according to claim 4, wherein the cavity (10) filled with the gel (9) is covered with a protective foil (12) in the area of the pressure- taking surface (11).

6. A pressure-measuring device according to one of the claims 1 to 5, wherein the first housing component (31) is provided with two locking recesses (37, 38) into which locking projections (40, 41) can be inserted, said locking projections (40, 41) being connected to the hose body (42).

7. A pressure-measuring device according to one of the claims 1 to 6, wherein the first housing component (31) is provided with a Luer-compatible hole (24) through which an adjacent side of the pressure sensor (8) communicates with the atmosphere.

8. A pressure-measuring device for invasive blood pressure measurement comprising:
a housing component (100; 200);
a pressure sensor (114) arranged on said housing component (100; 200);
a pressure-transmitting means (112) extending between said pressure sensor (114) and a pressure-taking surface (110) in said housing component (100; 200); and
a hose body (216) filled with a fluid for transmitting pressure from a pressure-measuring area;
characterized in that
the housing component (100; 200) is provided with an insertion opening (102; 202) and with a hose-holding area (104; 204), the hose body (216) being adapted to be inserted into said hose-holding area (104; 204) through said insertion opening (102; 202) and one wall of said hose body (216) being held in contact with the pressure-taking surface (110) when said hose body (216) has been introduced in said hose-holding area (104; 204), and
that the hose-holding area (104; 204) is implemented as an elongate cavity in said housing component (100; 200), the insertion opening (102; 202) being arranged in a section of the elongate cavity sidewall surrounding the cavity, and that, when the hose body has been inserted in said cavity, the wall of the hose body (216) is held, by the elongate cavity sidewall which substantially surrounds the hose body (216) in the area of the pressure-taking surface (110), in contact with said pressure-taking surface (110) in such a way that pressure tranmission is largely independent of the volume expansibility of the hose body (216).

9. A pressure-measuring device according to claim 8, wherein the insertion opening (102; 202) is an elongate opening through which the hose body (216) can be introduced in the hose-holding area (104; 204) by means of a movement perpendicular to its longitudinal extension.

10. A pressure-measuring device according to claim 9, wherein the hose body (216) consists of a flexible material and wherein, in the direction perpendicular to its longitudinal extension, the insertion opening (102; 202) has an insertion cross-section which is smaller than the diameter of the hose body (216).

11. A pressure-measuring device according to claim 10, wherein the cross-sectional area of the elongate cavity corresponds essentially to the cross-sectional area of the hose body (216).

12. A pressure-measuring device according to claim 10, wherein the elongate sidewall of the cavity is flat in the area where the pressure-taking surface (110) is arranged.

13. A pressure-measuring device according to one of the claims 10 to 12, wherein, starting from the inner housing component wall delimiting the cavity, the insertion opening (102; 202) is expanded outwards through the wall of said housing component (100; 200).

14. A pressure-measuring device according to one of the claims 8 to 13, wherein the pressure-transmitting means (112) is formed by a gel-filled cavity of the housing component (100; 200), said cavity extending from the pressure-taking surface (110) to the pressure sensor (114).

15. A pressure-measuring device according to one of the claims 8 to 14, wherein the housing component (200, 206) is provided with two locking recesses (208, 210) into which locking projections (212, 214) can be inserted, said locking projections being connected to the hose body (216).

16. A pressure-measuring device according to one of the claims 8 to 15, wherein the housing component (100; 200) is provided with a Luer-compatible hole (120) through which an adjacent side of the pressure sensor (114) communicates with the atmosphere.

17. A pressure-measuring device according to one of the claims 8 to 16, wherein the hose body (216) has a connection means arranged thereon, which substantially closes the insertion opening (202) when the hose body has been introduced in the hose-holding area (204).

18. A pressure-measuring device according to one of the claims 1 to 17, wherein the hose body (13; 216) has a substantially round cross-section.

19. A pressure-measuring device according to one of the claims 1 to 17, wherein the hose body (13; 216) has a substantially oval cross-section.

20. A pressure-measuring device according to one of the claims 1 to 17, wherein the hose body (13; 216) has a substantially polygonal cross-section.

21. A pressure-measuring device according to one of the claims 1 to 20, wherein, in the area where it abuts on the pressure-taking surface (11; 110), the hose body (13; 216) has a cross-sectional area which is larger than the rest of its cross-sectional area.

22. A pressure-measuring device according to one of the claims 1 to 20, wherein, in the area where it abuts on the pressure-taking surface (11; 110), the hose body (13; 216) has a flat wall portion (15).

23. A pressure-measuring device according to one of the claims 1 to 22, wherein the hose body (13; 216) extends in one piece up to an open hose end where the fluid filling of said hose body is in pressure communication with the blood pressure-measuring area when the blood pressure is being measured.

24. A pressure-measuring device according to claim 23, wherein the hose body (13; 216) is provided with a vent valve at its distal end which is located remote from the open end of the hose.

25. A pressure-measuring device according to one of the claims 1 to 24, wherein one end of the hose body (42; 216) is provided with a flushing system body (49; 226).

## Revendications

1. Dispositif de mesure de pression pour la mesure invasive de la pression sanguine, aux caractéristiques suivantes :
une première partie de boîtier (2) ;
un capteur de pression (8) disposé sur la première partie de boîtier (2) ;
un dispositif de transmission de pression (9, 10, 12) s'étendant entre le capteur de pression (8) et une face recevant la pression (11) dans la première partie de boîtier (2) ;
un élément de flexible (13) rempli d'un liquide pour la transmission de la pression à partir d'une zone de mesure de pression ; et
une seconde partie de boîtier (4), pouvant être fixée sur la première partie de boîtier (2), qui maintient l'élément de flexible (13), en vue d'une transmission de pression, en appui contre la face recevant la pression (11),
caractérisé par le fait
que la première partie de boîtier (2) présente un premier évidement guide-flexible (6) qui s'étend de part et d'autre de la face recevant la pression (11), la seconde partie de boîtier (4) présentant un second évidement guide-flexible (7) et le premier et le second évidement guide-flexible entourant sensiblement le flexible dans la zone de la face recevant la pression lorsque la seconde partie de boîtier (4) est fixée sur la première partie de boîtier, de sorte que la transmission de pression est largement indépendante de l'aptitude à la dilatation du volume de l'élément de flexible (13).

2. Dispositif de mesure de pression suivant la revendication 1, dans lequel la seconde partie de boîtier (4) est fixée à la première partie de boîtier (2) à l'aide d'une ou de plusieurs charnières (3).

3. Dispositif de mesure de pression suivant la revendication 2, dans lequel la seconde partie de boîtier (4) est maintenue en une position rabattue contre la première partie de boîtier (2) autour de la charnière (3) par un mécanisme de fermeture (5).

4. Dispositif de mesure de pression suivant l'une des revendications 1 à 3, dans lequel le dispositif de transmission de pression est constitué par un espace creux (10) de la première partie de boîtier (2), rempli d'un gel (9),.s'étendant de la face recevant la pression (11) au capteur de pression (8).

5. Dispositif de mesure de pression suivant la revendication 4, dans lequel l'espace creux (10) rempli de gel (9) est revêtu, dans la zone de la face recevant la pression (11), d'un film protecteur (12).

6. Dispositif de mesure de pression suivant l'une des revendications 1 à 5, dans lequel la première partie de boîtier (31) présente deux évidements d'encliquetage (37, 38) dans lesquels peuvent être engagés les doigts d'encliquetage (40, 41) qui sont assemblés à l'élément de flexible (42).

7. Dispositif de mesure de pression suivant l'une des revendications 1 à 6, dans lequel la première partie de boîtier (31) présente un alésage (24) compatible avec Luer par l'intermédiaire duquel un côté adjacent du capteur de pression (8) est en contact avec l'atmosphère.

8. Dispositif de mesure de pression pour la mesure invasive de la pression sanguine, aux caractéristiques suivantes :
un élément de boîtier (100 ; 200) ;
un capteur de pression (114) disposé sur l'élément de boîtier (100 ; 200) ;
un dispositif de transmission de pression (112) s'étendant entre le capteur de pression (114) et une face recevant la pression (110) dans l'élément de boîtier (100 ; 200) ; et
un élément de flexible (216) rempli d'un liquide pour la transmission de la pression à partir d'une zone de mesure de pression,
caractérisé par le fait
que l'élément de boîtier (100 ; 200) présente une ouverture d'introduction (102 ; 202) et une zone support de flexible (104 ; 204), l'élément de flexible (216) pouvant être introduit dans la zone support de flexible (104 ; 204) à travers l'ouverture d'introduction (102 ; 202) et étant maintenu, par une paroi de l'élément de flexible (216), en appui contre la face recevant la pression (110) lorsqu'il est introduit dans la zone support de flexible (104 ; 204), et
que la zone support de flexible (104 ; 204) est réalisée sous forme d'espace creux dans l'élément de boîtier (100 ; 200), dans un segment de la paroi latérale allongée de l'espace creux entourant l'espace creux étant disposée l'ouverture d'introduction (102 ; 202), la paroi de l'élément de flexible (216) étant maintenue, par la paroi latérale allongée de l'espace creux entourant sensiblement l'élément de flexible (216) dans la zone de la face recevant la pression (110), en appui contre la face recevant la pression (110) lorsque l'élément de flexible est introduit dans l'espace creux, de sorte que la transmission de pression est largement indépendante de l'aptitude à la dilatation du volume de l'élément de flexible (216)..

9. Dispositif de mesure de pression suivant la revendication 8, dans lequel l'ouverture d'introduction (102 ; 202) est une ouverture oblonge dans laquelle l'élément de flexible (216) peut être introduite dans la zone support de flexible (104; 204), à l'aide d'un mouvement perpendiculaire à son extension longitudinale.

10. Dispositif de mesure de pression suivant la revendication 9, dans lequel l'élément de flexible (216) est en un matériau flexible et dans lequel la section d'introduction de l'ouverture d'introduction (102; 202) est, dans la direction perpendiculaire à l'extension allongée, plus petite que le diamètre de l'élément de flexible (216).

11. Dispositif de mesure de pression suivant la revendication 10, dans lequel la face de section de l'espace creux allongé correspond sensiblement à la face de section de l'élément de flexible (216).

12. Dispositif de mesure de pression suivant la revendication 10, dans lequel la paroi latérale allongée de l'espace creux est plane dans la zone où est disposée la face recevant la pression (110).

13. Dispositif de mesure de pression suivant l'une des revendications 10 à 12, dans lequel l'ouverture d'introduction (102 ; 202) est élargie de la paroi intérieure de l'élément de boîtier (100 ; 200) délimitant l'espace creux, à travers l'élément de boîtier (100 ; 200), vers l'extérieur.

14. Dispositif de mesure de pression suivant l'une des revendications 8 à 13, dans lequel le dispositif de transmission de pression (112) est constitué par un espace creux de l'élément de boîtier (100 ; 200), rempli d'un gel, s'étendant de la face recevant la pression (110) au capteur de pression (114).

15. Dispositif de mesure de pression suivant l'une des revendications 8 à 14, dans lequel l'élément de boîtier (200, 206) présente deux évidements d'encliquetage (208, 210) dans lesquels peuvent s'engager les doigts d'encliquetage (212, 214) assemblés à l'élément de flexible (216).

16. Dispositif de mesure de pression suivant l'une des revendications 8 à 15, dans lequel l'élément de boîtier (100 ; 200) présente un alésage (120) compatible avec Luer par l'intermédiaire duquel un côté adjacent du capteur de pression (114) est en contact avec l'atmosphère.

17. Dispositif de mesure de pression suivant l'une des revendications 8 à 16, dans lequel est disposé, sur l'élément de flexible (216), un dispositif de connexion qui obture sensiblement l'ouverture d'introduction (202) lorsque l'élément de flexible est introduit dans la zone support de flexible (204).

18. Dispositif de mesure de pression suivant l'une des revendications 1 à 17, dans lequel l'élément de flexible (13 ; 216) présente une section sensiblement ronde.

19. Dispositif de mesure de pression suivant l'une des revendications 1 à 17, dans lequel l'élément de flexible (13 ; 216) présente une section sensiblement ovale.

20. Dispositif de mesure de pression suivant l'une des revendications 1 à 17, dans lequel l'élément de flexible (13 ; 216) présente une section sensiblement polygonale.

21. Dispositif de mesure de pression suivant l'une des revendications 1 à 20, dans lequel l'élément de flexible (13 ; 216) présente, dans sa zone d'appui sur la face recevant la pression (11 ; 110), une face de section augmentée par rapport à sa face de section ailleurs.

22. Dispositif de mesure de pression suivant l'une des revendications 1 à 20, dans lequel l'élément de flexible (13 ; 216) présente, dans sa zone d'appui sur la face recevant la pression (11 ; 110), une zone de paroi (15) plane.

23. Dispositif de mesure de pression suivant l'une des revendications 1 à 22, dans lequel l'élément de flexible (13 ; 216) s'étend d'une seule pièce jusqu'à une extrémité de flexible ouverte à laquelle son remplissage de liquide se trouve, lors de la mesure de la pression sanguine, en communication de pression avec la zone de mesure de pression sanguine.

24. Dispositif de mesure de pression suivant la revendication 23, dans lequel l'élément de flexible (13 ; 216) est pourvu, à son extrémité distale éloignée de l'extrémité de flexible ouverte, d'une soupape de purge d'air.

25. Dispositif de mesure de pression suivant l'une des revendications 1 à 24, dans lequel une extrémité de l'élément de flexible (42 ; 216) est pourvue d'un élément de système de rinçage (49 ; 226).
